# EUROPEAN PATENT SPECIFICATION

(11) **EP 3 322 452 B1**
(45) Date of publication and mention of the grant of the patent: **17.04.2019**
(21) Application number: 16754312.3
(22) Date of filing: 13.07.2016
(51) Int. Cl.: A61L 2/10, A45D 40/00, A45D 34/00

(54) **DELIVERY DEVICE FOR A LIQUID OR PASTE PRODUCT**
ABGABEVORRICHTUNG FÜR EIN FLÜSSIGES ODER PASTÖSES PRODUKT
DISPOSITIF DE DISTRIBUTION POUR UN PRODUIT LIQUIDE OU PÂTEUX

(30) Priority: 14.07.2015 IT UB20152165
(43) Date of publication of application: 23.05.2018
(73) Proprietor: Costanza, Isacco, 11010 Introd (AO) (IT)
(72) Inventor: Costanza, Isacco, 11010 Introd (AO) (IT)
(74) Representative: Aprà, Mario
(86) International application number: PCT/IB2016/000997
(87) International publication number: WO 2017/009706

(56) References cited:
- US-A1- 2014 264 070

## Description

The present invention relates to a delivery device for a liquid or paste product.

More in particular, the present invention relates to a device to be used, preferably, in the cosmetic and body care sector, for delivering liquid or paste products, such as beauty creams, oils for body treatment or massage and the like.

There are currently known, at the state of the art, different types of delivery devices for liquid or paste products, used in the aforesaid technical sector of reference.

In particular, there are known distributors for cosmetic products, to be used in the field of facial and body treatments for a person, which allow the user to collect, once or several times during the treatment, a given quantity of product required to carry out the treatment.

Normally, said known delivery devices are also provided with specific dosing means, to allow the user to collect the amount of product required for the treatment being administered, thereby reducing possible waste.

However, said known delivery devices have a plurality of problems.

Firstly, said known delivery devices are not able to provide a suitable solution to the problem related to alteration of the product to be delivered, related to the presence of microorganisms, or to different of chemical-physical causes due, for example, to the action of light, to variations in temperature, to the presence of metals or oxidation phenomena. In fact it is noted how contact of the product (for example, a cream for cosmetic treatment) with the air causes the rapid occurrence of oxidative phenomena triggered, in particular, by the action of oxygen on the lipid fraction of the compound.

Evident signs of alteration of the product resulting from oxidation phenomena are, for example, a change in colour and odour thereof.

It must be noted that besides losing, completely or partly, their respective chemical-physical properties advantageous and useful for the treatment to be administered, products with these alterations can also be harmful for the health of the person receiving the treatment, causing local skin irritations or allergic reactions, in particular in the case of sensitive individuals.

Taking account of the aforesaid problems, at the state of the art, some solutions have been provided, such as the use of delivery devices that prevent exposure to light radiation (for example, through the use of tanks made of material that is opaque and preferably dark in colour), the use of manufacturing materials that do not contain metal residues (responsible for the occurrence of oxidation phenomena of the product), storage of the delivery devices in places not exposed to specific sources of heat and/or light, when not in use.

The document US 2014/264070 A1 discloses a delivery device for a liquid or paste product, comprising a container with a tank for said product, a cover for said container, said cover including a collection chamber for the product delivered following a manual operation by the user, who, upon removing the cover, uses the product thus delivered, and then re-closes the container with the same cover, wherein said device comprises:
- at least one LED light emitter means in the ultraviolet range of the spectrum, the light beam of which is emitted and directed on the inside of said chamber;
- electronic circuit means for the control and electric powering of said at lead one LED light emitter means and electric battery means connected to said electronic circuit control means;

- electronic timer means connected to said electronic circuit control means, for timing the electrical power to said at least one LED light emitter means;
- control means for the electrical shut-down of said electronic circuit control means,
so that when the user re-closes said container with the cover, said control means being activated, said electronic circuit means cause electrical power to be supplied to said at least one LED light emitter means for the length of time set using said timer means, during which time the beam of light performs an anti-bacterial action on the inside and on the walls of said chamber.

However, said known solutions do not in any event provide comprehensive solutions to all the aforesaid problems.

In particular, it must be noted how any residues of product, accumulated after previous deliveries in proximity, for example, to one or more delivery nozzles or in a collection chamber of the delivered product, can lead to accumulation of bacteria.

Therefore, when the user collects a given quantity of product required for the treatment, the quantity of product, completely integral, that is delivered is particularly exposed to bacterial contamination when during delivery, respectively collection, it comes into contact with said residues of product delivered previously.

This aspect is also further emphasized by the fact that, in numerous cases, the operations to sanitize said known delivery devices are not very easy to perform and do not allow an appreciable level of cleaning and sanitization of these devices to be achieved.

This inevitably leads to potential risks for the safety of the person receiving the treatment, in terms of localized skin irritations, or even infections, especially in sensitive individuals.

The use is also known, in the preparation of said products for cosmetic treatments or the like, of chemical agents that are anti-oxidant, preservative or able to provide for absorption of UV radiation.

It is nonetheless evident how the use of said chemical agents could be potentially harmful for the person receiving the treatment, besides leading to possible skin irritations, respectively allergic reactions, especially in sensitive individuals.

Therefore, it must be noted how the aforesaid problems of said known delivery devices, and in particular those related to the impossibility of appreciably reducing bacterial contamination, remain essentially unsolved.

At the state of the art it is known how light, when having a blue spectrum (range 631-668 THz - wavelength 450-475 nm), has appreciable antibacterial properties.

In this direction, there are currently known a plurality of technical applications (for example, in the disinfection of instruments) and therapeutic applications (for example, localized skin treatments) in which light in the blue spectrum is used for bactericidal purposes.

The present invention, in consideration of the above, starting from the notion of the aforesaid problems of the cited state of the art, intends to provide a solution to these problems.

An object of the present invention is to provide a delivery device for a liquid or paste product which is able to greatly reduce the risks of bacterial contamination of the product delivered, inside a collection chamber of this product. Moreover, an object of the present invention is also to provide a device as mentioned, which is able to allow the best preservation of the same product from oxidizing agents. Another object of the present invention is to provide a delivery device as indicated, which is easy for the user to sanitize, thereby allowing an appreciable level of cleaning and sterilization to be achieved.

A further object of the present invention is to provide a device as specified, which is simple for the user to use.

On the other hand, an object of the present invention is to provide a device as said, which has a simple structure, is easy to produce on an industrial scale with essentially limited costs.

In view of these objects, the present invention provides a delivery device for a liquid or paste product, the essential feature of which constitutes the subject-matter of claim 1. Further advantageous features are described in the subsequent dependent claims.

The aforesaid claims are intended as fully set forth herein.

In particular, according to the present invention, said delivery device for a liquid or paste product, comprising a container with a tank for said product, a cover for said container, said cover including a collection chamber for the product delivered following manual operation by the user, who, upon removing the cover, uses the product thus delivered, and then re-closes the container with the same cover, comprising:
- at least one LED light emitter means, the light beam of which is emitted and directed on the inside of said chamber;
- electronic circuit means for the control and electric powering of said at least one LED light emitter means and electric battery means connected to said electronic circuit control means;
- electronic timer means connected to said electronic circuit control means, for timing the electrical power to said at least one LED light emitter means;
- control means for the electrical shut-down of said electronic circuit control means, so that when the user re-closes said container with the cover, said control means being activated, said electronic circuit means cause electrical power to be supplied to said at least one LED light emitter means for the length of time set using said timer means, during which time the beam of light performs an anti-bacterial action on the inside and on the walls of said chamber, wherein said container comprises control means for controlling the quantity of liquid or paste product delivered from said tank into said chamber, where said tank is flexible and is in fluid communication with said chamber through a delivery nozzle, and compression means for said flexible tank controlled by means of a thrust device, movable inside said container, and driven manually from the outside of the same container, while a base plate closes the end of said container opposite said cover, and is movably mounted by rotation with respect to the same container, wherein the base plate and the container comprise means for one-directional rotation, and wherein said at least one LED light emitter means emits light in the blue range of the spectrum and wherein said one-directional rotation means are shaped with teeth and flexible fins, mutually opposed, which when rotating generate snaps perceptible to the user, corresponding to advancing steps of said plate inside said container, for the dosage of said product delivered into said chamber.

The present invention will be more apparent from the detailed description below, with reference to the accompanying drawing, of a delivery device for a liquid or paste product, wherein:
- Fig. 1 is a vertical sectional view of a delivery device for a liquid or paste product, according to an example of embodiment of the invention, with the tank expanded;
- Fig. 2 is a view similar to Fig. 1, but with the tank compressed;
- Fig. 3 is a view similar to Fig. 1, but with parts in an exploded view and with the tank omitted;
- Fig. 4 is a sectional detail view on a larger scale of the detail of a communication conduit and of a delivery nozzle of the device of Fig. 1, wherein said delivery nozzle is in a water-tight closed condition, not in use;
- Fig. 5 is a view similar to Fig. 4, but in which said delivery nozzle is in an open condition in use to allow discharge of said product;
- Fig. 6 is a sectional detail view on a larger scale of the detail of a toothed projection in a position engaged in a blind groove on a container, for dismountable connection of a base plate to said container;
- Fig. 7 is a view similar to Fig. 6, but in which said toothed projection is in a position disengaged from said groove;
- Fig. 8 is a sectional view along the line VIII-VIII of Fig. 1.

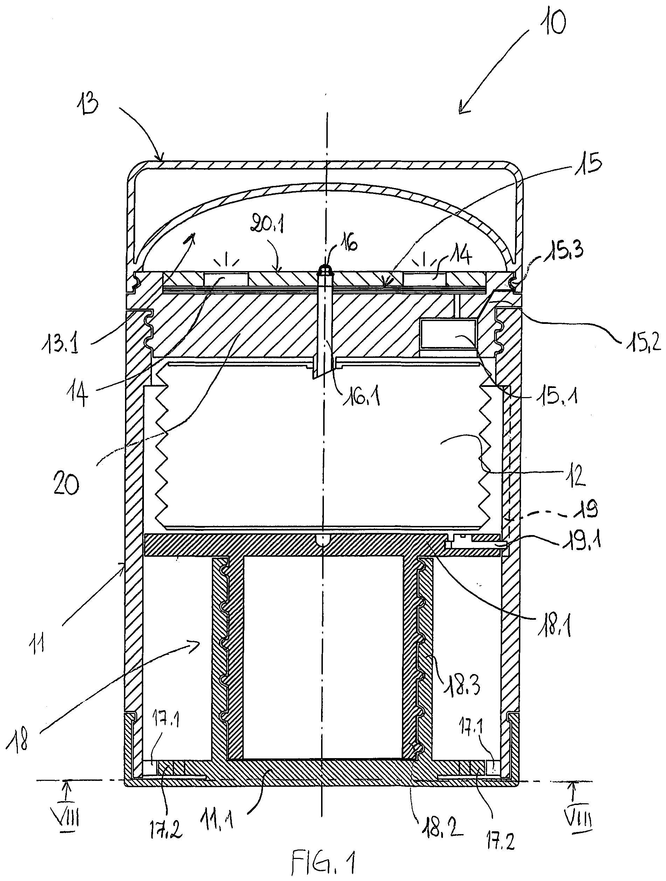

In the aforesaid Figs. 1 to 3, the numeral 10 indicates, as a whole, a delivery device for a liquid or paste product, according to an example of embodiment of the present invention.

Said device 10 comprises, according to the prior art, a container 11 with a tank 12 for said product, a cover 13 for said container 11, said cover 13 including a collection chamber 13.1 for the product delivered following manual operation by the user, who, upon removing the cover 13, uses the product thus delivered, and then re-closes the container 11 with the same cover 13.

In particular, according to the invention, said device comprises (Figs. 1-3):
- at least one LED (light emitting diode) light emitter means 14 in the blue range of the spectrum, the light beam of which is emitted and directed on the inside of said chamber 13.1;
- electronic circuit means 15 for the control and electric powering of said at lead one LED light emitter means 14 and electric battery means 15.1 connected to said electronic circuit control means 15;
- electronic timer means (not shown) connected to said electronic circuit control means 15, for timing the electrical power to said at least one LED light emitter means 14;
- control means 15.2 for the electrical shut-down of said electronic circuit control means 15.

In this way, when the user re-closes said container 11 with the cover 13, said control means 15.2 being activated by said cover 13, said electronic circuit means 15 cause electrical power to be supplied to said at least one LED light emitter means 14 for the length of time set using said timer means, during which time the beam of light performs an anti-bacterial action on the inside and on the walls of said chamber 13.1. Therefore, upon subsequent delivery, the risk of bacterial contamination of the product thus delivered and collected in said chamber 13.1 is greatly reduced, thereby overcoming the aforesaid problems of the prior art.

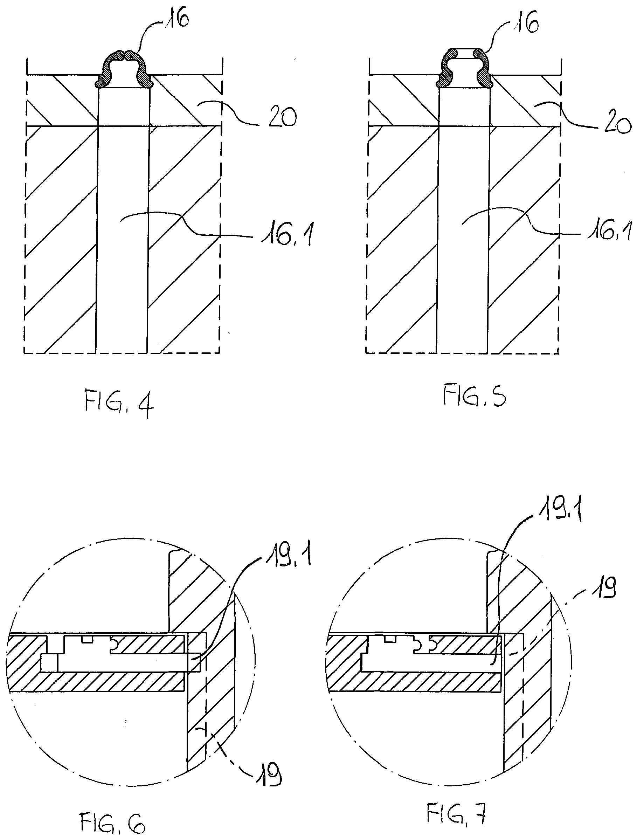

In the embodiment illustrated, a plurality of LED light emitter means 24 are arranged on a printed microcircuit board 15, which includes said circuit means for the control and said electronic timer means, electrically connected to electric battery means 15.1, housed in a base plate 20, engaged in a dismountable manner at the top of the container 11, and which receives in screw coupling said cover 13, when closed. Advantageously, in the embodiment illustrated (Figs. 4 and 5) said container 11 encloses a tank 12 of a liquid or paste product, communicating with said chamber 13.1 through a delivery nozzle 16, that is water-tight when not in use (Fig. 4). Said tank 12 selectively delivers, through said nozzle 16 when in use (Fig. 5), a quantity of liquid or paste product lower than the maximum capacity of said chamber 13.1.

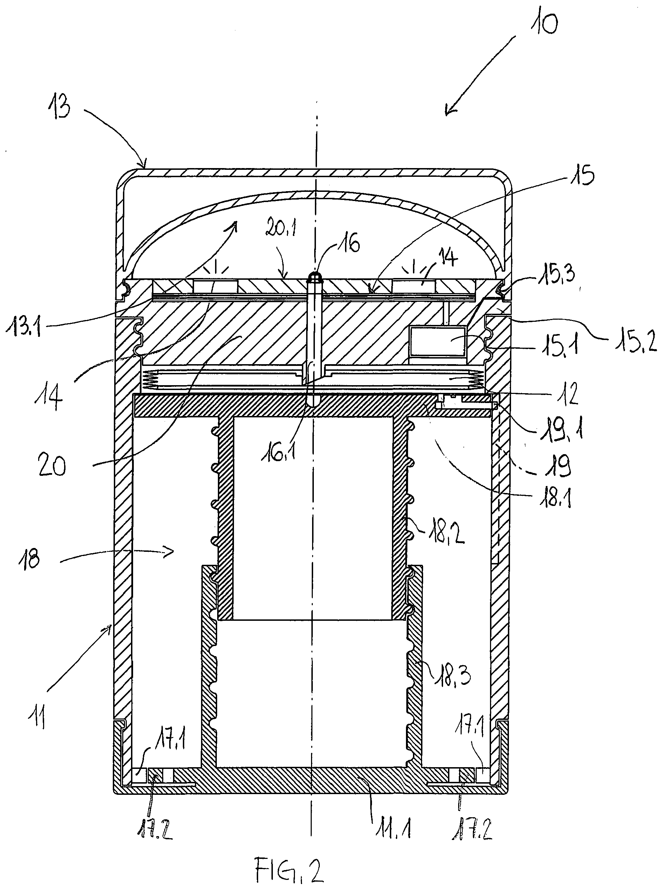

In Fig. 1, the tank 12 is illustrated in a completely filled condition, while in Fig. 2 the tank 12 is illustrated in an almost completely empty condition.

According to the present embodiment of the invention (Figs. 1 and 2), said container 11 comprises control means 17 for controlling the quantity of liquid or paste product delivered from said tank 12 into said chamber 13.1. Said tank 12 is flexible and arranged in fluid communication with said chamber 13.1 through said nozzle 16. Compression means 18 for said flexible tank 12 are controlled by means of a thrust device 18.1, movable inside said container 11, and driven manually from the outside of the same container.

In this way, it is very easy for the user to determine delivery of said product, contained in said tank 12.

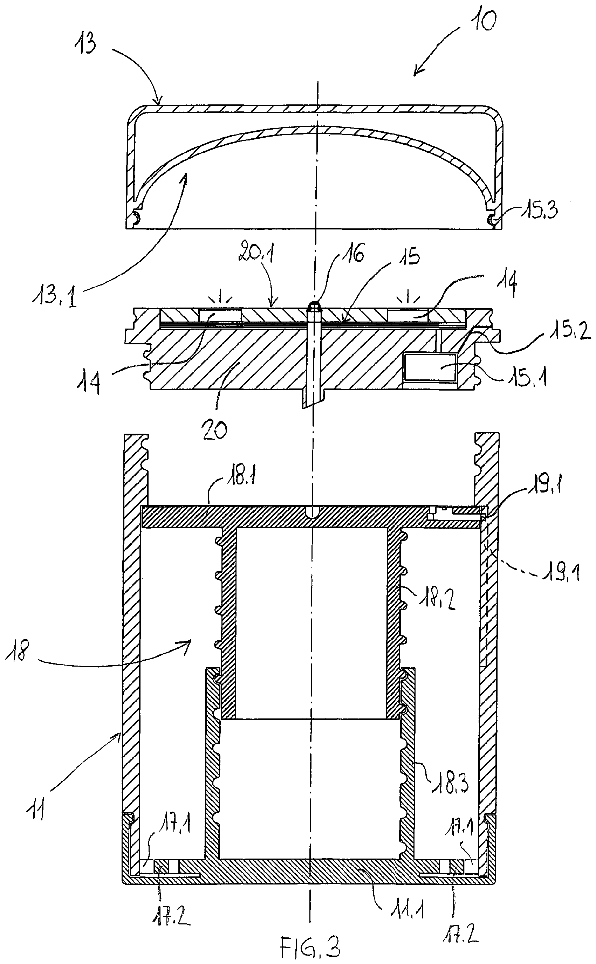

More in particular, in the embodiment illustrated (Figs. 1 and 3) said compression means 18 for said flexible tank 12 are controlled by means of a thrust plate 18.1, movable in an axial direction from a bottom part of said container 11 towards said cover 13, a translation mechanism for said thrust plate 18.1 with a screw 18.2 and nut 18.3, one part of which is associated with said plate 18.1, and joined in axial movement to the container 11, and the other part of which is associated with a base plate 11.1 that closes the end of said container 11 opposite said cover 13, and is movably mounted by rotation with respect to the same container 11.

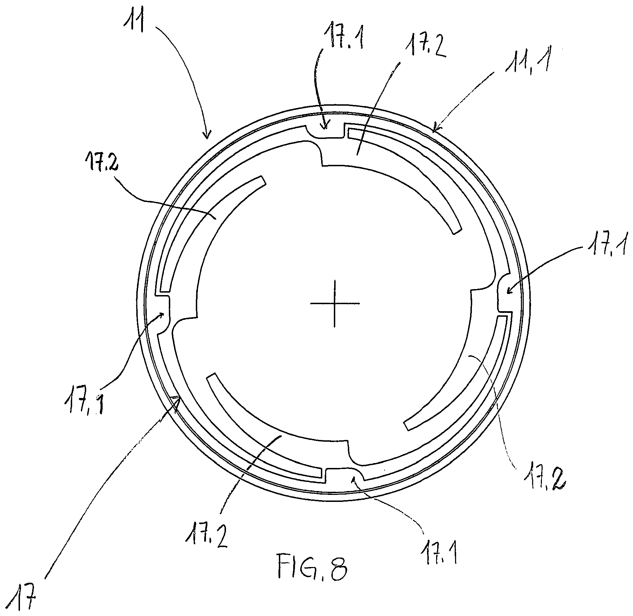

On the other hand, according to the present invention, said base plate 11.1 and said container 11 comprise means 17 for the one-directional rotation of the base plate 11.1 with respect to the container 11, that causes the advancement of said thrust plate 18.1 along said container 11 (in particular, see Fig. 8).

More in particular, in the embodiment illustrated (Fig. 8), said one-directional rotation means 17 are shaped as a plurality of teeth 17.1 and a plurality of flexible fins 17.2, mutually opposed, which when rotating generate snaps perceptible to the user, corresponding to advancing steps of said plate 18.1 inside said container 11, for the dosage of said product delivered into said chamber 13.1.

In this way, the user is able to determine delivery of the required quantity of product, greatly reducing the risk of waste.

In the present embodiment of the invention, said flexible tank 12 is interchangeable in said container 11.

Therefore, the user can, with simple operations, replace said tank 12 with another tank containing the same or another product, according to need.

Conveniently, according to the present embodiment of the invention, said device 10 comprises the following parts, mutually assembled in a dismountable manner (Figs. 1 to 3):
- said container 11 with a substantially tubular shape;
- said base plate 11.1, associated with said nut 18.3;
- said plate 18.1 associated with said screw 18.2 and with a toothed projection 19 engaged in a blind groove 19.1 on said container 11, and selectively retractable from the container 11, for the relative disengagement of the plate 18.1 from the container 11 (Figs. 6 and 7);
- said base plate 20 associated with the inside of the end of said container 11 next to said cover 13, which provides at least one wall 20.1 of said chamber 13.1, on which faces said at least one LED light emitter device 14, and supporting said delivery nozzle 16 and a respective fluid communication conduit 16.1 between said nozzle 16 and said tank 12, as well as
- said electronic circuit means 15 for the control and electric powering of said at least one LED light emitter means 14 and electric battery means 15.1 connected to said electronic circuit means 15, supported by means of said base plate 20;
- said electronic timer means connected to said electronic circuit means 15, for timing the electrical power to said at least one LED light emitter means 14, and
- said control means 15.2 for the electrical shut-down of said electronic circuit control means 15, and
- said cover 13 which provides the remaining part of said chamber 13.1, in a manner complementary to said base plate 20, and automatic activation means 15.3 for said control means 15.2 for the electrical shut-down of said electronic circuit means 15.

The aforesaid dismountable characteristics of the device 10 allow improved cleaning and sanitizing thereof to be achieved, in a manner that also is very easy for the user.

On the other hand, for the same reasons, operations to perform maintenance on the device 10, or to replace any parts thereof in case of damage or depletion of the content of the tank 12, are also very easy to carry out.

Although the delivery nozzle 16 is illustrated in the form of a flexible water-tight valve with calibrated bore, which opens under fluid pressure and automatically closes when said pressure ceases, other embodiments of said nozzle are also possible according to the present invention.

As is evident based on the considerations above, said delivery device 10 for a liquid or paste product is capable of providing a noteworthy reduction in the risk of bacterial contamination of the product that is delivered, in said collection chamber.

Moreover, said device 10 as mentioned is capable of allowing the best possible preservation of the same product from oxidizing agents.

In addition, said device 10 as indicated can be easily cleaned and sanitized by the user, thereby achieving an appreciable level of cleanliness and sterilization.

Further, said device 10 as specified is simple for the user to use.

On the other hand, said device 10 as said has a simple structure that is easy to produce on an industrial scale and with essentially limited costs.

As can be seen from the above, the present invention allows the objects set forth in the introduction to be achieved in a simple and advantageous manner.

## Claims

1. Delivery device (10) for a liquid or paste product, comprising a container (11) with a tank (12) for said product, a cover (13) for said container (11), said cover (13) including a collection chamber (13.1) for the product delivered following manual operation by the user, who, upon removing the cover (13), uses the product thus delivered, and then re-closes the container (11) with the same cover (13), comprising:
- at least one LED light emitter means (14), the light beam of which is emitted and directed on the inside of said chamber (13.1);
- electronic circuit means (15) for the control and electric powering of said at least one LED light emitter means (14) and electric battery means (15.1) connected to said electronic circuit control means (15);
- electronic timer means connected to said electronic circuit control means (15), for timing the electrical power to said at least one LED light emitter means (14);
- control means (15.2) for the electrical shut-down of said electronic circuit control means (15), so that when the user re-closes said container (11) with the cover (13), said control means (15.2) being activated, said electronic circuit means (15) cause electrical power to be supplied to said at least one LED light emitter means (14) for the length of time set using said timer means, during which time the beam of light performs an anti-bacterial action on the inside and on the walls of said chamber (13.1), wherein said container (11) comprises control means (17) for controlling the quantity of liquid or paste product delivered from said tank (12) into said chamber (13.1), where said tank (12) is flexible and is in fluid communication with said chamber (13.1) through a delivery nozzle (16), and compression means (18) for said flexible tank (12) controlled by means of a thrust device (18.1), movable inside said container (11), and driven manually from the outside of the same container, while a base plate (11.1) closes the end of said container (11) opposite said cover (13), and is movably mounted by rotation with respect to the same container (11), wherein the base plate and the container comprise means for one-directional rotation, wherein said at least one LED light emitter means (14) emits light in the blue range of the spectrum and wherein said one-directional rotation means (17) are shaped with teeth (17.1) and flexible fins (17.2), mutually opposed, which when rotating generate snaps perceptible to the user, corresponding to advancing steps of said plate (18.1) inside said container (11), for the dosage of said product delivered into said chamber (13.1).

2. Device (10) according to claim 1, **characterized in that** said container (11) encloses a tank (12) of liquid or paste product, communicating with said chamber (13.1) through said delivery nozzle (16) that is water-tight when not in use, **and in that said** tank (12) selectively delivers, through said nozzle (16) when in use, a quantity of liquid or paste product lower than the maximum capacity of said chamber (13.1).

3. Device (10) according to claim 1, **characterized in that** said compression means (18) for said flexible tank (12) are controlled by means of a thrust plate (18.1), movable in an axial direction from a bottom part of said container (11) towards said cover (13), a translation mechanism for said thrust plate (18.1) with a screw (18.2) and nut (18.3), one part of which is associated with said plate (18.1), and joined in axial movement to the container (11), and the other part of which is associated with said base plate (11.1).

4. Device (10) according to claim 3, **characterized in that** said base plate (11.1) and said container (11) comprise means (17) for the one-directional rotation of the base plate (11.1) with respect to the container (11), that causes the advancement of said thrust plate (18.1) along said container (11).

5. Device (10) according to one or more of the previous claims, **characterized in that** said flexible tank (12) is interchangeable in said container (11).

6. Device (10) according to one or more of the previous claims, **characterized in that** it comprises the following parts, mutually assembled in a dismountable manner:
- said container (11) with a substantially tubular shape;
- said base plate (11.1), associated with said nut (18.3);
- said plate (18.1) associated with said screw (18.2) and with a toothed projection (19) engaged in a blind groove (19.1) on said container (11), and selectively retractable from the container (11), for the relative disengagement of the plate (18.1) from the container (11);
- a base plate (20) associated with the inside of the end of said container (11) next to said cover (13), and providing at least one wall (20.1) of said chamber (13.1), on which faces said at least one LED light emitter device (14) in the blue range of the spectrum, and supporting said delivery nozzle (16) and a respective fluid communication conduit (16.1) between said nozzle (16) and said tank (12), as well as
- said at least one LED light emitter means (14);
- said electronic circuit means (15) for the control and electric powering of said at least one LED light emitter means (14) and electric battery means (15.1) connected to said electronic circuit means (15), supported by means of said base plate (20) ;
- said electronic timer means connected to said electronic circuit means (15), for timing the electrical power to said at least one LED light emitter means (14), and
- said control means (15.2) for the electrical shut-down of said electronic circuit control means (15), and
- said cover (13) which provides the remaining part of said chamber (13.1), in a manner complementary to said base plate (20), and automatic activation means (15.3) for said control means (15.2) for the electrical shut-down of said electronic circuit means (15) .

7. Device (10) according to claim 6, **characterized in that** said base plate (20) has said at least one LED light emitter device (14) located in said at least one wall (20.1) of said chamber (13.1).

## Patentansprüche

1. Eine Spendervorrichtung (10) für ein flüssiges oder dickflüssiges Produkt, umfassend einen Behälter (11) mit Speicher (12) für besagtes Produkt, einen Deckel (13) für besagten Behälter (11), wobei besagter Deckel (13) eine Sammelkammer (13.1) von dem nach manueller Kontrolle vonseiten des Bedieners gespendeten Produkt umfasst, der, nach Entfernung des Deckels (13), das so gespendete Produkt benutzt und daraufhin den Behälter (11) mit dem Deckel (13) selbst wieder verschließt, umfassend:
- mindestens eine Ausstrahlungsvorrichtung (14) von LED-Licht, dessen Lichtstrahl im Inneren besagter Kammer (13.1) ausgestrahlt und orientiert wird;
- elektronische Schaltvorrichtungen (15) zur Kontrolle und elektrischen Speisung besagter mindestens einer Ausstrahlungsvorrichtung (14) von LED-Licht und der elektrischen Batterievorrichtungen (15.1), die mit besagten elektronischen Kontroll-Schaltvorrichtungen (15) verbunden sind;
- elektronische Schaltuhrenvorrichtungen, verbunden mit besagten elektronischen Kontroll-Schaltvorrichtungen (15), für die Zeitsteuerung der elektrischen Speisung von besagter mindestens einer Ausstrahlungsvorrichtung (14) von LED-Licht;
- Kontrollvorrichtungen (15.2) der elektrischen Unterbrechung von besagten elektronischen Kontroll-Schaltvorrichtungen (15), sodass, wenn der Benutzer den besagten Behälter (11) mit dem Deckel (13) wieder verschließt, und die besagten Kontrollvorrichtungen (15.2) aktiviert werden, besagte elektronische Schaltvorrichtungen (15) die elektrische Speisung von besagter mindestens einer Ausstrahlungsvorrichtung (14) von LED-Licht für die mittels besagter Schaltuhrenvorrichtungen voreingestellte Zeit auslösen, dessen Lichtstrahl im Inneren und an den Wänden besagter Kammer (13.1) eine antibakterielle Wirkung ausübt, wobei besagter Behälter (11) Kontrollvorrichtungen (17) der Menge an aus besagtem Speicher (12) in besagter Kammer (13.1) gespendetem flüssigem oder dickflüssigem Produkt umfasst, wobei besagter Speicher (12) flexibel ist und in Fluidverbindung mit besagter Kammer (13.1) angeordnet ist mittels einer Spenderdüse (16), sowie Kompressionsvorrichtungen (18) von besagtem flexiblem Speicher (12), kontrolliert mittels eines Druckorgans (18.1), beweglich im Inneren besagten Behälters (11) und manuell von außerhalb des selbigen Behälters betätigt, während eine Abschlusskappe (11.1) das besagten Deckels (13) gegenüberliegende Ende besagten Behälters (11) verschließt und rotationsbeweglich montiert ist in Bezug auf den Behälter (11) selbst, wobei die Abschlusskappe und der Behälter Vorrichtungen zur unidirektionalen Rotation umfassen, wobei mindestens eine Ausstrahlungsvorrichtung (14) von LED-Licht Licht in der Frequenz des blauen Spektrums ausstrahlt und wobei besagte Vorrichtungen zur unidirektionalen Rotation (17) in der Form von Zähnen (17.1) und flexiblen Rippen (17.2) gestaltet sind, die gegenseitig gegenüberstehen und eine Rotation in vom Bediener wahrnehmbare Stufen auslösen, die Vorschubschritten von besagter Tellerscheibe (18.1) in besagtem Behälter (11) entsprechen, zur Dosierung von besagtem in besagter Kammer (13.1) gespendetem Produkt.

2. Eine Vorrichtung (10) gemäß Anspruch 1, **dadurch gakennzeichnet, dass** besagter Behälter (11) einen Speicher (12) von flüssigem oder dickflüssigem Produkt umfasst, der mit besagter Kammer (13.1) durch besagte in nicht-operativem Zustand flüssigkeitsdichte Spenderdüse (16) in Verbindung steht, und **dadurch, dass** besagter Speicher (12) durch besagte Düse (16) in operativem Zustand eine Menge von flüssigem oder dickflüssigem Produkt, geringer als die maximale Kapazität besagter Kammer (13.1), selektiv spendet.

3. Eine Vorrichtung (10) gemäß Anspruch 1, **dadurch gekennzeichnet, dass** besagte Kompressionsvorrichtungen (18) von besagtem flexiblem Speicher (12) kontrolliert werden durch eine Schiebertellerscheibe (18.1), die in axialer Richtung von einem Endbereich besagten Behälters (11) in Richtung des Deckels (13) beweglich ist, durch einen Übertragungsmechanismus von besagter Schiebertellerscheibe (18.1) in Schrauben- (18.2) und Spindelmutterform (18.3), von der ein Teil mit besagter Tellerscheibe (18.1) verbunden ist, in der axialen Verschiebung in Bezug auf den Behälter (11) festgelegt, und dessen anderes Teil mit besagter Abschlusskappe (11.1) verbunden ist.

4. Eine Vorrichtung (10) gemäß Anspruch 3, **dadurch gekennzeichnet, dass** besagte Abschlusskappe (11.1) und besagter Behälter (11) Vorrichtungen zur unidirektionalen Rotation (17) der Abschlusskappe (11.1) selbst in Bezug auf den Behälter (11) umfasst, dem der Vorschub von besagter Tellerscheibe (18.1) längs besagten Behälters (11) entspricht.

5. Eine Vorrichtung (10) gemäß einem oder mehreren der vorhergehenden Ansprüche, **dadurch gekennzeichnet, dass** der besagte flexible Speicher (12) in besagtem Behälter (11) austauschbar ist.

6. Eine Vorrichtung (10) gemäß einem oder mehreren der vorhergehenden Ansprüche, **dadurch gekennzeichnet, dass** sie die folgenden Teile umfasst, gegenseitig auf zerlegbare Weise zusammengebaut:
- besagter Behälter (11) mit im Wesentlichen schlauchförmiger Form;
- besagte Abschlusskappe (11.1), verbunden mit besagter Spindelmutter (18.3);
- besagte Tellerscheibe (18.1), verbunden mit besagter Schraube (18.2) und mit Zahnhervorhebungen (19), die in einer Blindnut (19.1) von besagtem Behälter (11) engagiert sind und selektiv in Bezug auf den Behälter (11) selbst eingezogen werden können zur relativen Befreiung der Tellerscheibe (18.1) vom Behälter (11);
- eine Basis (20), verbunden im Inneren des Endes von besagtem Behälter (11) nahe dem besagten Deckel (13), die für mindestens eine Wand (20.1) von besagter Kammer (13.1) sorgt, auf der besagte mindestens eine Ausstrahlungsvorrichtung (14) von LED-Licht mit Licht in der Frequenz des blauen Spektrums geht, und die besagte Spenderdüse (16) und eine dazugehörige Leitung (16.1) zur Verbindung der Flüssigkeit zwischen besagter Düse (16) und besagtem Speicher (12) stützt, sowie
- besagte mindestens eine Ausstrahlungsvorrichtung (14) von LED-Licht;
- besagte elektronische Schaltvorrichtungen (15) zur Kontrolle und elektrischen Speisung besagter mindestens einer Ausstrahlungsvorrichtung (14) von LED-Licht und der elektrischen Batterievorrichtungen (15.1), die mit besagten elektronischen Kontroll-Schaltvorrichtungen (15) verbunden sind und mittels besagter Basis (20) gestützt sind;
- besagte elektronische Schaltuhrenvorrichtungen, verbunden mit besagten elektronischen Kontroll-Schaltvorrichtungen (15), für die Zeitsteuerung der elektrischen Speisung von besagter mindestens einer Ausstrahlungsvorrichtung (14) von LED-Licht, und
- besagte Kontrollvorrichtungen (15.2) der elektrischen Unterbrechung von besagten elektronischen Kontroll-Schaltvorrichtungen (15), und
- besagter Deckel (13), der für den restlichen Teil besagter Kammer (13.1) auf komplementäre Weise in Bezug auf die Basis (20) sorgt, und Vorrichtungen zur automatischen Aktivierung (15.3) von besagten Kontrollvorrichtungen (15.2) der elektrischen Unterbrechung von besagten elektronischen Schaltvorrichtungen (15).

7. Eine Vorrichtung (10) gemäß Anspruch 6, **dadurch gekennzeichnet, dass** besagte Basis (20) besagte mindestens eine Ausstrahlungsvorrichtung (14) von LED-Licht aufweist, angeordnet in besagter mindestens einer Wand (20.1) besagter Kammer (13.1).

## Revendications

1. Dispositif (10) distributeur pour un produit liquide ou pâteux, comprenant un récipient (11) avec réservoir (12) pour ledit produit, un couvercle (13) dudit récipient (11), ledit couvercle (13) incluant une chambre (13.1) de collecte du produit distribué à la suite d'un contrôle manuel de la part de l'utilisateur qui, après retrait du couvercle (13), utilise le produit ainsi distribué, refermant ensuite le récipient (11) avec le couvercle (13) même, comprenant:
- au moins un moyen émetteur (14) de lumière LED dont le faisceau lumineux est émis et orienté à l'intérieur de ladite chambre (13.1);
- des moyens de circuits électroniques (15) de contrôle et alimentation électrique dudit au moins un moyen émetteur (14) de lumière LED et des moyens à batterie électrique (15.1) connectés auxdits moyens de circuits (15) électroniques de contrôle;
- des moyens électroniques temporisés connectés par rapport auxdits moyens de circuits (15) électroniques de contrôle, pour la temporisation de l'alimentation électrique dudit au moins un moyen émetteur (14) de lumière LED;
- des moyens de contrôle (15.2) de la fermeture électrique desdits moyens de circuits (15) électroniques de contrôle, de sorte que lorsque l'utilisateur referme ledit récipient (11) avec le couvercle (13), lesdits moyens de contrôle (15.2) étant activés, lesdits moyens de circuits (15) électroniques provoquent l'alimentation électrique dudit au moins un moyen émetteur (14) de lumière LED pendant le temps prédéfini à l'aide desdits moyens temporisés, dont le faisceau lumineux exerce une action antibactérienne à l'intérieur et sur les parois de ladite chambre (13.1), où ledit récipient (11) comprend des moyens de contrôle (17) de la quantité de produit liquide ou pâteux distribuée par ledit réservoir (12) dans ladite chambre (13.1), où ledit réservoir (12) est flexible et placé en communication de fluide avec ladite chambre (13.1) à travers une buse de distribution (16), et des moyens de compression (18) dudit réservoir (12) flexible commandés à l'aide d'un organe de poussée (18.1), mobile à l'intérieur dudit récipient (11), et actionné manuellement par l'extérieur du récipient même, tandis qu'un fond (11.1) ferme l'extrémité dudit récipient (11) opposée audit couvercle (13), et il est monté de manière mobile par rotation par rapport au récipient (11) même, où le fond et le récipient comprennent des moyens de rotation unidirectionnelle, où ledit au moins un moyen émetteur (14) de lumière LED émet de la lumière dans la fréquence du spectre bleu et où lesdits moyens de rotation unidirectionnelle (17) sont conformés en guise de dents (17.1) et ailettes flexibles (17.2), réciproquement opposées, et provoquent une rotation par crans perceptibles par l'opérateur, correspondants à des pas d'avance dudit plateau (18.1) dans ledit récipient (11), pour le dosage dudit produit distribué dans ladite chambre (13.1).

2. Dispositif (10) selon la revendication 1, **caractérisé par le fait que** ledit récipient (11) contient un réservoir (12) de produit liquide ou pâteux, communiquant avec ladite chambre (13.1) à travers ladite buse (16) de distribution étanche au fluide en condition non opérationnelle, **et par le fait que** ledit réservoir (12) distribue sélectivement, à travers ladite buse (16) en condition opérationnelle, une quantité de produit liquide ou pâteux inférieure à la contenance maximale de ladite chambre (13.1).

3. Dispositif (10) selon la revendication 1, **caractérisé par le fait que** lesdits moyens de compression (18) dudit réservoir (12) flexible sont commandés à l'aide d'un plateau pousseur (18.1), mobile dans la direction axiale d'une partie de fond dudit récipient (11) vers ledit couvercle (13), un mécanisme de translation dudit plateau pousseur (18.1) à vis (18.2) et vis-mère (18.3), dont une partie est associée audit plateau (18.1), contraint par déplacement axial par rapport au récipient (11), et dont l'autre partie est associées audit fond (11.1).

4. Dispositif (10) selon la revendication 3, **caractérisé par le fait que** ledit fond (11.1) et ledit récipient (11) comprennent des moyens de rotation unidirectionnelle (17) du fond (11.1) même par rapport au récipient (11), à laquelle correspond l'avance dudit plateau (18.1) le long dudit récipient (11) .

5. Dispositif (10) selon une ou plusieurs des revendications précédentes, **caractérisé par le fait que** ledit réservoir (12) flexible est interchangeable dans ledit récipient (11).

6. Dispositif (10) selon une ou plusieurs des revendications précédentes, **caractérise par le fait qu'**il comprend les parties suivantes, réciproquement assemblées de manière démontable:
- ledit récipient (11) de forme substantiellement tubulaire;
- ledit fond (11.1), associé à ladite vis-mère (18.3);
- ledit plateau (18.1) associé à ladite vis (18.2) et présentant une proéminence dentée (19) engagée dans une rainure borgne (19.1) dudit récipient (11), et sélectivement rétractible par rapport au récipient (11) même, pour le dégagement relatif du plateau (18.1) par rapport au récipient (11);
- une base (20) associée à l'intérieur de l'extrémité dudit récipient (11) à proximité dudit couvercle (13), laquelle fournit au moins une paroi (20.1) de ladite chambre (13.1), sur laquelle se présente ledit au moins un dispositif émetteur (14) de lumière LED dans le spectre de lumière bleue, et soutient ladite buse (16) de distribution et un conduit respectif (16.1) de communication de fluide entre ladite buse (16) et ledit réservoir (12), ainsi que
- ledit au moins un moyen émetteur (14) de lumière LED;
- lesdits moyens de circuits (15) électronique de contrôle et alimentation électrique dudit au moins un moyen émetteur (14) de lumière LED et des moyens à batterie (15.1) électrique raccordés auxdits moyens de circuits (15) électroniques, soutenus par ladite base (20);
- lesdits moyens électroniques temporisés connectés par rapport auxdits moyens de circuits (15) électroniques, pour la temporisation de l'alimentation électrique dudit au moins un moyen émetteur (14) de lumière LED, et
- lesdits moyens de contrôle (15.2) de la fermeture électrique desdits moyens de circuits (15) électroniques de contrôle, et
- ledit couvercle (13) lequel fournit la partie restante de ladite chambre (13.1), de manière complémentaire par rapport à ladite base (20), et des moyens d'activation automatique (15.3) desdits moyens de contrôle (15.2) de la fermeture électrique desdits moyens de circuits (15) électroniques.

7. Dispositif (10) selon la revendication 6, **caractérisé par le fait que** ladite base (20) présente ledit au moins un dispositif émetteur (14) de lumière LED disposé dans ladite au moins une paroi (20.1) de ladite chambre (13.1).
